# EUROPEAN PATENT SPECIFICATION

(11) **EP 2 477 680 B1**
(45) Date of publication and mention of the grant of the patent: **01.01.2014**
(21) Application number: 10752590.9
(22) Date of filing: 15.09.2010
(51) Int. Cl.: A61M 5/145, A61M 5/315

(54) **MEDICAMENT ADMINISTRATION DEVICE**
FESTKÖRPER ZUR DOSISKONTROLLE
UNITÉ DE VERROUILLAGE ET DE CONTRÔLE À INTÉGRER DANS UN APPAREIL MÉDICAL

(30) Priority: 18.09.2009 EP 09170720
(43) Date of publication of application: 25.07.2012
(73) Proprietor: Sanofi-Aventis Deutschland GmbH, 65929 Frankfurt (DE)
(72) Inventor: NAGEL, Thomas, 01737 Tharandt (DE); RICHTER, René, 01737 Tharandt (DE); WITT, Robert, 01159 Dresden (DE)
(86) International application number: PCT/EP2010/063516
(87) International publication number: WO 2011/032962

(56) References cited:
- US-A- 4 313 439
- US-A- 4 424 057

## Description

### Technical Field

The invention relates to a medicament administration device comprising a rigid medicament container for a liquid medicament with an outlet for the medicament.

### Background of the Invention

Many medicaments have to be injected into the body. This applies in particular to medicaments, which are deactivated or have their efficiency remarkably decreased by oral administration, e.g. proteines (such as insulin, growth hormones, interferons), carbohydrates (e.g. heparin), antibodies and the majority of vaccines. Such medicaments are predominantly injected by means of syringes, medicament pens or medicament pumps.

Some medicaments have to be administered by inhaling them from so called inhalers.

US 4 313 439 A discloses a system for the administration of a medicament to a patient in small, controlled doses over an extended period in response to a continuously generated force. The force may be maintained continuously on a reservoir of the medicament In intermittent communication with a site in the body of the patient through a flexible and compressible tube. Alternatively the force may be applied intermittently to the reservoir through the action of an escapement mechanism.

US 4 424 057 A discloses a wet-dry syringe for combining and mixing a liquid and a solid medicament or at least two dissimilar liquid medicaments prior to the application thereof to a patient. The syringe comprises a first vial containing a liquid or solid medicament and a second vial containing a liquid medicament. The second vial has an end seal including a hollow piercing needle to pierce a first end seal of the first vial causing medicament to flow from the second vial into the first vial thereby mixing the medicaments prior to application to a patient by means of a needle piecing assembly which pierces a second end seal of the first vial and a patient. The second vial functions as a piston rod and aids in the discharge of the medicaments.

### Summary of the invention

It is an object of the present invention to provide an improved medicament administration device.

The object is achieved by a medicament administration device according to claim 1.

Preferred embodiments of the invention are given in the dependent claims.

A medicament administration device according to the invention comprises a rigid medicament container for a liquid medicament with an outlet for the medicament and a storage means for storing solid particles. In addition it comprises solid particles storable in the storage means and a feeding means for feeding solid particles from the storage means into the medicament container to displace a dose of medicament by solid particles, thereby squeezing the dose of medicament through the outlet of the medicament container.

The storage means and the feeding means allow for dispensing a dose of medicament by displacing the dose by solid particles. In this way the medicament can be dosed very accurately by feeding a definite amount of solid particles into the medicament container.

In a preferred embodiment of the medicament administration device the solid particles are incompressible. This allows for an easy medicament dosing as the incompressibility of the solid particles implies that the total volume of the solid particles fed into the medicament container determines directly the dose of medicament displaced by these solid particles. In particular, using solid particles of equal definite volume, the number of solid particles fed into the medicament container is directly proportional to the dose of medicament displaced by these solid particles.

Preferably the solid particles consist of a material which is inert to chemical reactions with the liquid medicament. This avoids advantageously that the chemical structure of the medicament is affected by the solid particles fed into the medicament container. In particular there is no need to separate the solid particles from the medicament, in contrast to other displacement media such as media used with hydraulic systems. Furthermore chemically inert materials avoid that the volume of the solid particles fed into the medicament container is affected by chemical reactions with the medicament and thus that the dose of medicament displaced by solid particles is affected by such chemical reactions.

In particular the solid particles may consist of glass and/or a rubber material and/or polytetrafluoroethylene. These materials are chemically inert to chemical reactions with many medicaments and are therefore appropriate to a number of medicaments.

Furthermore the solid particles may be ball-shaped or cubical-shaped. This has the advantage that the total volume of solid particles fed into the medicament container can be easily determined from their number and thus allows for an easy medicament dosing. Furthermore ball-shaped or cubical-shaped solid particles do not easily get stuck, in contrast to solid particles with a more complicated shape which may cause catching or jamming of solid particles. Hence ball-shaped or cubical-shaped particles can be more easily stored and fed into the medicament container than solid particles with a more complicated shape.

Preferably the storage means comprises subunits for storing separately portions of solid particles. This allows for preparing medicament doses by correspondingly sizing the subunits. E.g., one may use subunits of equal size that corresponds to a minimum medicament dose to be dispensed, or a fraction thereof. Alternatively one may use subunits of different size such that any needed dose corresponds to a combination of subunits.

The feeding means may comprise a piston to press solid particles out of the storage means. Furthermore the feeding means may comprise connection means connecting the storage means and the medicament container to feed solid particles from the storage means into the medicament container. This allows to feed solid particles into the medicament container through the connection means by using the piston to exert pressure to the storage means.

Preferably the medicament administration device comprises a restraining means to prevent solid particles from leaving the medicament container. The restraining means may comprise a filter for the outlet of the medicament container, the filter being impenetrable for the solid particles. Such a restraining means avoids advantageously that solid particles are dispensed together with a medicament dose. Alternatively, one may use a physical separation method, based, for instance, on a gravitational of centrifugal force, to prevent solid particles from leaving the medicament container.

The medicament container may be part of an injection arrangement or an inhaler arrangement for delivering a liquid medicament to a human or an animal.

The injection arrangement may comprise a valve and a hollow needle for piercing a patient's skin, the valve and needle being arranged at the outlet of the medicament container. In that case the outlet of the medicament container may comprise an interface for receiving a hollow injection needle. Alternatively, the needle may be integrated with the medicament container. In case of a jet injector, instead of the needle, a jet nozzle may be arranged.

The medicament container may preferably be used for delivering one of an analgesic, an anticoagulant, insulin, insulin derivate, heparin, Lovenox, a vaccine, a growth hormone, a peptide hormone, a protein, antibodies and complex carbohydrates. Further scope of applicability of the present invention will become apparent from the detailed description given hereinafter. However, it should be understood that the detailed description and specific examples, while indicating preferred embodiments of the invention, are given by way of illustration only.

### Brief Description of the Drawings

The present invention will become more fully understood from the detailed description given hereinbelow and an accompanying drawing which is given by way of illustration only, and thus is not limitive of the present invention, and wherein:

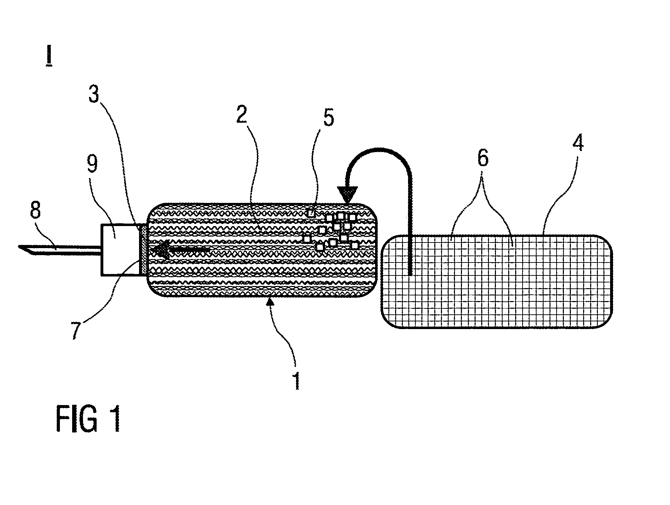

Figure 1 illustrates a medicament administration device comprising a medicament container and storage means from which solid particles are fed into the medicament container.

### Detailed Description of Preferred Embodiments

Figure 1 illustrates a medicament administration device 1 according to the invention which is part of an injection arrangement I for delivering a liquid medicament. The medicament administration device 1 comprises a rigid medicament container 2 with an outlet 3 for the liquid medicament and a storage means 4 from which solid particles 5 are fed into the medicament container 2. The storage means 4 comprises subunits 6 for storing separately portions of solid particles 5.

The medicament administration device 1 further comprises a filter 7 covering the outlet 3 of the medicament container 2. The filter 7 is penetrable for the liquid medicament, but impenetrable for the solid particles 5. The filter 7 thus restrains the solid particles 5 from leaving the medicament container 2.

The injection arrangement I further comprises a hollow needle 8 for piercing a patient's skin. The needle 8 is connected to the outlet 3 so that the liquid medicament can flow from the interior of the medicament container 2 through the outlet 3 to the needle 8. The injection arrangement I further comprises an interface 9 for attaching the needle 8 to the outlet 3.

Alternatively the medicament injection arrangement I may be designed as a jet injector having a jet nozzle instead of a needle 8.

The solid particles 5 are cubical-shaped, equally sized and incompressible. They consist of a material which is inert to chemical reactions with the liquid medicament. In particular they may consist of the same material as the medicament container 2 which is typically made of glass. However, other appropriate materials may also be used, such as a rubber material or polytetrafluoroethylene.

The solid particles 5 are fed from the storage means 4 into the medicament container 2 to displace a medicament dose by solid particles 5, thereby squeezing the medicament dose through the outlet 3 of the medicament container 2. The medicament dose is adjusted by the amount of solid particules 5 fed into the medicament container 2. This amount is adjusted through the subunits 6.

In one embodiment all subunits 6 contain an equal number of solid particles 5 and this number represents the minimum medicament dose to be dispensed by means of the medicament administration device 1. Any higher dose dispensable by means of the medicament administration device 1 is in that case a multiple of the minimum dose and is dispensed by feeding the solid particles 5 of a corresponding number of subunits 6 into the medicament container 2, either consecutively or at once.

In an alternative embodiment the number of solid particles 5 stored in the subunits 6 varies over the subunits 6 such that any needed medicament dose corresponds to a subunit 6 or a combination of subunits 6 with an appropriate number of solid particles 5.

In a third embodiment the subunits 6 are adjusted individually to the needs of a particular patient.

The medicament container 1 may preferably be used for delivering one of an analgesic, an anticoagulant, insulin, insulin derivate, heparin, Lovenox, a vaccine, a growth hormone, a peptide hormone, a protein, antibodies and complex carbohydrates.

The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(02)25, Asp28] Exendin-4(1-39),
des Pro36 [Met(O) 14 Trp(02)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(O2)25, IsoAsp2B] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2, des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2, des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2, des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2, des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2, H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

### List of References

- 1: medicament administration device
- 2: medicament container
- 3: outlet
- 4: storage means
- 5: solid particles
- 6: subunit
- 7: filter
- 8: needle
- 9: interface
- l: injection arrangement

## Claims

1. Medicament administration device (1) comprising a rigid medicament container (2) for a liquid medicament with an outlet (3) for the medicament, a storage means (4) for storing solid particles (5), solid particles (5) storable in the storage means (4), and a feeding means for feeding solid particles (5) from the storage means (4) into the medicament container (2) to displace a dose of medicament by solid particles (5), thereby squeezing the dose of medicament through the outlet (3) of the medicament container (2).

2. Medicament administration device (1) according to claim 1.
**characterized in that** the solid particles (5) are incompressible.

3. Medicament administration device (1) according to claim 1 or 2,
**characterized in that** the solid particles (5) consist of a material which is inert to chemical reactions with the liquid medicament.

4. Medicament administration device (1) according to any one of claims 1 to 3,
**characterized in that** the solid particles (5) consist of glass and/or a rubber material and/or polytetrafluoroethylene.

5. Medicament administration device (1) according to any one of claims 1 to 4,
**characterized in that** the solid particles (5) are ball-shaped or cubical-shaped.

6. Medicament administration device (1) according to any one of the preceding claims,
**characterized in that** the storage means (4) comprises subunits (6) for storing separately portions of solid particles (5).

7. Medicament administration device (1) according to any one of the preceding claims,
**characterized in that** the feeding means comprises a piston to press solid particles (5) out of the storage means (4).

8. Medicament administration device (1) according to any one of the preceding claims,
**characterized in that** the feeding means comprise connection means connecting the storage means (4) and the medicament container (2) to feed solid particles (5) from the storage means (4) into the medicament container (2).

9. Medicament administration device (1) according to any one of the preceding claims,
**characterized by** a restraining means to prevent solid particles (5) from leaving the medicament container (2).

10. Medicament administration device (1) according to claim 9,
**characterized in that** the restraining means comprises a filter (7) for the outlet (3) of the medicament container (2), the filter (7) being impenetrable for the solid particles (5).

11. Injection arrangement (I) for delivering a liquid medicament comprising a medicament administration device (1) according to any one of the claims 1 to 10.

12. Injection arrangement (I) according to claim 11,
**characterized in that** a valve and a hollow needle (8) for piercing a patients skin are arranged at the outlet (3) of the medicament container (2).

13. Inhaler arrangement for delivering a liquid medicament comprising a medicament administration device (1) according to any one of the claims 1 to 10.

## Patentansprüche

1. Medikamentenverabreichungseinheit (1), umfassend einen starren Medikamentenbehälter (2) für ein flüssiges Medikament mit einer Auslassöffnung (3) für das Medikament, ein Aufbewahrungsmittel (4) zum Aufbewahren fester Partikel (5), feste Partikel (5), die in dem Aufbewahrungsmittel (4) aufbewahrbar sind, und ein Zuführungsmittel zum zuführen fester Partikel (5) aus dem Aufbewahrungsmittel (4) in den Medikamentenbehälter (2), um eine Dosis an Medikament durch feste Partikel (5) zu verdrängen und so die Dosis an Medikament durch die Auslassöffnung (3) des Medikamentenbehälters (2) zu drücken.

2. Medikamentenverabreichungseinheit (1) gemäß Anspruch 1, **dadurch gekennzeichnet, dass** die festen Partikel (5) nicht komprimierbar sind.

3. Medikamentenverabreichungseinheit (1) gemäß Anspruch 1 oder 2, **dadurch gekennzeichnet, dass** die festen Partikel (5) aus einem Material bestehen, das gegen chemische Reaktionen mit dem flüssigen Medikament inert ist.

4. Medikamentenverabreichungseinheit (1) gemäß einem der Ansprüche 1 bis 3, **dadurch gekennzeichnet, dass** die festen Partikel (5) aus Glas und/oder einem Gummimaterial und/oder Polytetrafluorethylen bestehen.

5. Medikamentenverabreichungseinheit (1) gemäß einem der Ansprüche 1 bis 4, **dadurch gekennzeichnet, dass** die festen Partikel (5) kugelförmig oder würfelförmig sind.

6. Medikamentenverabreichungseinheit (1) gemäß einem der vorstehenden Ansprüche,
**dadurch gekennzeichnet, dass** das Aufbewahrungsmittel (4) Untereinheiten (6) zum getrennten Aufbewahren von Portionen fester Partikel (5) umfasst.

7. Medikamentenverabreichungseinheit (1) gemäß einem der vorstehenden Ansprüche,
**dadurch gekennzeichnet, dass** das Zuführungsmittel einen Kolben zum Drücken fester Partikel (5) aus dem Aufbewahrungsmittel (4) umfasst.

8. Medikamentenverabreichungseinheit (1) gemäß einem der vorstehenden Ansprüche,
**dadurch gekennzeichnet, dass** das Zuführungsmittel Verbindungsmittel umfasst, das das Aufbewahrungsmittel (4) mit dem Medikamentenbehälter (2) verbindet, um dem Medikamentenbehälter (2) feste Partikel (5) aus dem Aufbewahrungsmittel (4) zuzuführen.

9. Medikamentenverabreichungseinheit (1) gemäß einem der vorstehenden Ansprüche,
**gekennzeichnet durch** ein Rückhaltemittel, um feste Partikel (5) am Austreten aus dem Medikamentenbehälter (2) zu hindern.

10. Medikamentenverabreichungseinheit (1) gemäß Anspruch 9,
**dadurch gekennzeichnet, dass** das Rückhaltemittel ein Filter (7) für die Auslassöffnung (3) des Medikamentenbehälters (2) umfasst, wobei das Filter (7) für die festen Partikel (5) undurchlässig ist.

11. Injektionseinrichtung (I) zum Abgeben eines flüssigen Medikaments, umfassend eine Medikamentenverabreichungseinheit (1) gemäß einem der Ansprüche 1 bis 10.

12. Injektionseinrichtung (I) gemäß Anspruch 11,
**dadurch gekennzeichnet, dass** an der Auslassöffnung (3) des Medikamentenbehälters (2) ein Ventil und eine Hohlnadel (8) zum Durchstechen der Haut eines Patienten angeordnet sind.

13. Inhalatoreinrichtung zum Abgeben eines flüssigen Medikaments, umfassend eine Medikamentenverabreichungseinheit (1) gemäß einem der Ansprüche 1 bis 10.

## Revendications

1. Dispositif d'administration de médicament (1) comprenant un récipient de médicament rigide (2) pour un médicament liquide avec une sortie (3) pour le médicament, un moyen de stockage (4) pour stocker des particules solides (5), des particules solides (5) pouvant être stockées dans le moyen de stockage (4), et un moyen d'alimentation pour alimenter les particules solides (5) depuis le moyen de stockage (4) dans le récipient de médicament (2) pour déplacer une dose de médicament par les particules solides (5), en comprimant la dose de médicament à travers la sortie (3) du récipient de médicament (2).

2. Dispositif d'administration de médicament (1) selon la revendication 1,
**caractérisé en ce que** les particules solides (5) sont incompressibles.

3. Dispositif d'administration de médicament (1) selon la revendication 1 ou 2,
**caractérisé en ce que** les particules solides (5) sont constituées d'un matériau qui est inerte vis-à-vis des réactions chimiques avec le médicament liquide.

4. Dispositif d'administration de médicament (1) selon l'une quelconque des revendications 1 à 3, **caractérisé en ce que** les particules solides (5) sont constituées de verre et/ou d'un matériau en caoutchouc et/ou de polytétrafluoroéthylène.

5. Dispositif d'administration de médicament (1) selon l'une quelconque des revendications 1 à 4,
**caractérisé en ce que** les particules solides (5) sont en forme de billes ou de cubes.

6. Dispositif d'administration de médicament (1) selon l'une quelconque des revendications précédentes,
**caractérisé en ce que** le moyen de stockage (4) comprend des sous-unités (6) pour stocker séparément des portions de particules solides (5).

7. Dispositif d'administration de médicament (1) selon l'une quelconque des revendications précédentes,
**caractérisé en ce que** le moyen d'alimentation comprend un piston pour presser des particules solides (5) hors du moyen de stockage (4).

8. Dispositif d'administration de médicament (1) selon l'une quelconque des revendications précédentes,
**caractérisé en ce que** le moyen d'alimentation comprend un moyen de connexion connectant le moyen de stockage (4) et le récipient de médicament (2) pour alimenter des particules solides (5) depuis le moyen de stockage (4) jusque dans le récipient de médicament (2).

9. Dispositif d'administration de médicament (1) selon l'une quelconque des revendications précédentes,
**caractérisé par** un moyen de retenue pour empêcher que des particules solides (5) ne quittent le récipient de médicament (2).

10. Dispositif d'administration de médicament (1) selon la revendication 9,
**caractérisé en ce que** le moyen de retenue comprend un filtre (7) pour la sortie (3) du récipient de médicament (2), le filtre (7) étant impénétrable pour les particules solides (5).

11. Agencement d'injection (I) pour distribuer un médicament liquide comprenant un dispositif d'administration de médicament (1) selon l'une quelconque des revendications 1 à 10.

12. Agencement d'injection (I) selon la revendication 11,
**caractérisé en ce qu'**une soupape et une aiguille creuse (8) pour percer la peau d'un patient sont agencées au niveau d'une sortie (3) du récipient de médicament (2).

13. Agencement d'inhalateur pour distribuer un médicament liquide comprenant un dispositif d'administration de médicament (1) selon l'une quelconque des revendications 1 à 10.
